(19) Europäisches Patentamt
European Patent Office
Office européen des brevets

(11) **EP 2 247 646 B2**

(12) **NOUVEAU FASCICULE DE BREVET EUROPEEN**
Après la procédure d'opposition

(45) Date de publication et mention de la décision concernant l'opposition:
**08.03.2023 Bulletin 2023/10**

(45) Mention de la délivrance du brevet:
**08.07.2015 Bulletin 2015/28**

(21) Numéro de dépôt: **09710616.5**

(22) Date de dépôt: **03.02.2009**

(51) Classification Internationale des Brevets (IPC):
**C08G 69/06** *(2006.01)*    **C08G 69/16** *(2006.01)*
**C08G 69/28** *(2006.01)*    **C08J 3/12** *(2006.01)*
**C08L 77/00** *(2006.01)*    **A61K 8/04** *(2006.01)*
**A61K 8/02** *(2006.01)*    **A61K 8/88** *(2006.01)*
**A61K 9/16** *(2006.01)*    **A61K 47/34** *(2006.01)*
**A61Q 1/12** *(2006.01)*    **C09D 5/03** *(2006.01)*
**C09D 177/00** *(2006.01)*    **C09D 5/08** *(2006.01)*
**A61Q 1/08** *(2006.01)*    **C08G 69/08** *(2006.01)*
**C08G 69/26** *(2006.01)*    **C09D 7/65** *(2018.01)*
**C09D 7/40** *(2018.01)*

(52) Classification Coopérative des Brevets (CPC):
**C08J 3/12; A61K 8/0245; A61K 8/88; A61K 47/34; A61Q 1/12; C08G 69/08; C08G 69/26; C09D 5/08; C09D 7/65; C09D 7/69; G06Q 30/02; G06Q 50/188;** A61K 9/1694; A61K 2800/412; A61K 2800/652;

(Cont.)

(86) Numéro de dépôt international:
**PCT/FR2009/050160**

(87) Numéro de publication internationale:
**WO 2009/101320 (20.08.2009 Gazette 2009/34)**

(54) **POUDRE FINE DE POLYAMIDE ISSU DE MATIERES RENOUVELABLES ET PROCEDE DE FABRICATION D'UNE TELLE POUDRE**

FEINES POLYAMIDPULVER AUS ERNEUERBAREN MATERIALIEN UND VERFAHREN ZUR HERSTELLUNG EINES SOLCHEN PULVERS

FINE POWDER OF POLYAMIDE FROM RENEWABLE MATERIALS AND METHOD FOR MAKING SUCH POWDER

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **15.02.2008 FR 0850994**
**07.04.2008 US 4285608 P**

(43) Date de publication de la demande:
**10.11.2010 Bulletin 2010/45**

(60) Demande divisionnaire:
**15170091.1 / 2 949 314**

(73) Titulaire: **ARKEMA FRANCE**
**92700 Colombes (FR)**

(72) Inventeurs:
• **LOYEN, Karine**
**F-27500 Pont-audemer (FR)**

• **LABONNE, Eric**
**F-27300 Bernay (FR)**

(74) Mandataire: **Bandpay & Greuter**
**30, rue Notre-Dame des Victoires**
**75002 Paris (FR)**

(56) Documents cités:
DE-A1- 4 421 454    JP-A- 2004 051 751
US-A- 4 069 184    US-A- 4 927 860
US-A- 6 146 762    US-A1- 2006 127 424

• **OLIVIER LECOQ: "Etude de la broyabilité de différents matériaux pulvérulents à l'aide d'un test d'impact à jet d'air" MÉMOIRE DE THÈSE, [Online] 19 décembre 1997 (1997-12-19), pages 1-162, XP002497047 Université de Technologie de Compiègne Extrait de l'Internet: URL:http://perso.enstimac.fr/~lecoq/These_OLe.pdf> [extrait le 2008-09-23]**

- **DATABASE WPI Thomson Scientific, London, GB; AN 1993-131378 XP002497048 -& JP 05 070598 A (DAICEL HUELS KK) 23 mars 1993 (1993-03-23)**
- **ANONYME: "FICHE DE DONNÉES DE SÉCURITÉ: RILSAN POUDRES COSMÉTIQUES" ARKEMA, 14 novembre 2007 (2007-11-14), pages 1-7, XP002497046**

(52) Classification Coopérative des Brevets (CPC): (Cont.)
C08J 2377/00; C08L 77/00

**Description**

Domaine de l'invention

La présente invention se rapporte aux poudres fines, telles que celles utilisées en cosmétique, pharmacie ou parfumerie. La présente invention concerne plus particulièrement une poudre fine de polyamide issu de matières renouvelables. L'invention a également pour objet un procédé de fabrication d'une telle poudre issue de matières premières renouvelables.

Dans la cosmétique conventionnelle, on trouve essentiellement des ingrédients d'origine pétrolière ou d'origine synthétique. Leurs procédés d'obtention sont parfois considérés comme polluants sur l'environnement.

En effet, les matières premières utilisées pour la synthèse de ces ingrédients sont obtenues par vapocraquage ou craquage catalytique de coupes pétrolières. L'utilisation de ces matières contribue à l'augmentation de l'effet de serre. Etant donnée la diminution des réserves pétrolières mondiales, la source de ces matières premières va peu à peu s'épuiser.

Les matières premières issues de la biomasse sont de source renouvelable et ont un impact réduit sur l'environnement. Elles ne nécessitent pas toutes les étapes de raffinage (très coûteuses en énergie) des produits pétroliers. La production de CO2 est réduite de sorte qu'elles contribuent moins au réchauffement climatique.

Il apparaît donc nécessaire de disposer de procédés de synthèse non dépendants de matière première d'origine fossile, mais utilisant plutôt des matières premières d'origine renouvelable.

Aujourd'hui, les consommateurs sont de plus en plus attirés par les produits d'origine végétale qui ont la réputation d'être plus sûrs et plus compatibles pour la peau.

Par ailleurs, sur un marché aussi concurrentiel que celui des cosmétiques, les formulateurs doivent répondre à la demande des consommateurs pour des cosmétiques associant efficacité, texture innovante, et qualités sensorielles. Or ces propriétés dépendent à la fois des matières premières et des procédés utilisés.

De plus, l'émergence des produits cosmétiques pour homme, et qui permettent de répondre à leurs attentes et exigences propres, nécessite également la recherche de nouvelles matières premières plus adaptées.

La présente invention a donc pour but de fournir une poudre polymère, qui répond aux différentes exigences énoncées ci-dessus, à la fois en terme d'efficacité, de texture, de qualités sensorielles et en terme d'éthique écologique et biologique.

Technique antérieure

A titre d'exemples de poudres couramment utilisées en cosmétique, on peut citer celles d'origine minérale (talc, silice, kaolin, sericite, carbonate de calcium ou de magnesium) ; les oxydes (TiO2, ZnO) ; celles d'origine végétale (l'amidon) ; celles d'origine animale (poudre de soie) ; et celles d'origine synthétique : polyméthacrylate de méthyle (PMMA), polyamide 12 (PA 12).

Les poudres d'origine minérale présentent des propriétés sensorielles inférieures à celles des poudres synthétiques. Leur toucher est souvent rugueux et sec. Elles peuvent assécher la peau et donner lieu à des irritations.

Les poudres de Polyamide 12, bien que d'origine pétrolière, sont particulièrement appréciées en cosmétique pour le toucher soyeux et doux caractéristique qu'elles confèrent aux formulations. Le PA 12 se rencontre aussi bien dans les formulations de maquillage (ombres à paupières, fond de teint, rouge à lèvre, mascara...), les formulations de soin (crèmes de jour, crème de nuit, lait corporel), les formulations de produits solaires, etc. Pour obtenir les propriétés sensorielles requises pour ces formulations, les poudres doivent avoir une granulométrie moyenne inférieure à 100 $\mu$m, de préférence inférieure à 50 $\mu$m, et de préférence encore inférieure à 20 $\mu$m.

A titre d'exemple de poudre PA 12 pour formulation cosmétique, on peut citer des produits commerciaux : Orgasol 2002 EXD NAT COS (ARKEMA), Nylonpoly WL 10 (Création Couleurs), Covabead N12-10 (LCW), SP500 (TORAY), UBESTA (UBE), Tegolon 12-20 (Evonik).

Différents procédés de fabrication permettent de produire des poudres de polyamide 12, et conduisent chacun à des caractéristiques de poudre différentes.

On peut citer par exemple, les procédés de synthèse directs, qui conduisent à des poudres de polyamide 12 par polymérisation de lauryl lactame ou d'acide amino dodécanoïque. En fonction du type de procédé, il est possible d'obtenir des poudres parfaitement sphériques non poreuses ou bien des poudres sphéroïdales poreuses. Dans ce dernier cas, on peut citer les poudres de PA 12, commercialisées par ARKEMA France sous le nom ORGASOL®.

Il existe par ailleurs des procédés de dissolution/précipitation qui conduisent à des poudres de polymère par dissolution d'un polymère dans un solvant, puis reprécipitation sous forme de poudre. Ce type de procédé conduit à des poudres sphéroïdales, de porosité variable.

Par exemple, la demande de brevet DE4421454 décrit un procédé de synthèse de poudre polyamide 12 par dissolution et précipitation, le but de l'invention dans cette demande étant d'obtenir une poudre PA 12 de forme sphérique et de granulométrie resserrée. Le procédé utilisé requiert l'utilisation d'un polyamide de masse moléculaire et de viscosité suffisamment importantes pour permettre sa précipitation. Aussi, la poudre obtenue après précipitation a un diamètre tel qu'un broyage dans des conditions sévères est nécessaire pour obtenir une poudre fine de diamètre inférieur à 30 $\mu$m.

Le document US2006/127424 décrit un procédé comprenant la dissolution de polyamide, par exemple PA6 ou PA12, dans un solvant suivie de précipitation qui résulte en des poudres de forme régulière toutes en formes d'haltère.

Cependant, dans tous les cas cités précédemment, le lauryllactame et l'acide amino-dodécanoïque polymérisés pour obtenir ces poudres PA 12 sont issus de la pétrochimie. De plus, pour obtenir des poudres de granulométrie inférieure à 20 $\mu$m, ces procédés utilisent des quantités importantes de solvants, eux-mêmes issus de la pétrochimie.

A la différence des poudres de polyamide 12, les poudres de polyamide 11 (PA 11) sont fabriqués à partir de matières premières d'origine végétale.

Les matières végétales présentent l'avantage de pouvoir être cultivées en grande quantité, selon la demande, sur la majeure partie du globe terrestre et d'être renouvelables. Une matière première renouvelable est une ressource naturelle, animale ou végétale, dont le stock peut se reconstituer sur une période courte à l'échelle humaine. Il faut en particulier que ce stock puisse se renouveler aussi vite qu'il est consommé.

Le PA 11 est produit par la société Arkema. Il existe toute une gamme de produits à base de PA 11, sous le nom commercial de Rilsan® 11 ou Rilsan® B. La matière première de base de ces produits est l'huile de ricin (Castor oil, en anglais), extraite de la plante du même nom (le Ricin commun), à partir des graines de ricin. Classiquement, des poudres de PA 11 sont obtenues par un procédé de broyage d'un prépolymère, à l'aide d'un broyeur à impact Un tel broyeur à impact comprend un dispositif de rotor-stator qui effectue un broyage mécanique. Le rotor est doté de taquets ou marteaux, grâce auxquels le produit est projeté contre une voûte crantée formée par le stator. Selon ce procédé actuel, le produit est broyé par impact, chocs ou attrition et les particules de poudre obtenues sont de différentes formes plus ou moins anguleuses.

Cependant, les caractéristiques, notamment de granulométrie (généralement supérieure à 100 $\mu$m) et/ou de forme, de ces poudres de PA 11 de l'art antérieur ne permettent pas de les utiliser en tant que poudres cosmétiques avec des propriétés sensorielles adaptées aux applications maquillage et soin (telles que les crèmes de jour, crèmes de nuit, ou crèmes solaires). En particulier, il n'est pas possible avec le procédé industriel de fabrication de PA 11 utilisé aujourd'hui, d'obtenir des particules de poudre de diamètre moyen inférieur à 30 $\mu$m.

Les brevets EP1847559 et US6127513 décrivent des procédés permettant d'obtenir des poudres de polyamide, notamment de PA 11 ou 12, par synthèse directe ou par dissolution-précipitation, conduisant à des poudres sphériques, poreuses ou non, de granulométrie inférieure à 100 $\mu$m. Toutefois, ces poudres sont systématiquement obtenues en solvant, et elles ont donc un impact environnemental non négligeable.

Les poudres de PA 11 ou 12 de l'art antérieur, qu'elles soient issues d'un procédé de synthèse directe, d'un procédé de dissolution-précipitation ou bien issues d'un procédé de broyage d'un prépolymère, ne permettent donc pas de remplir toutes les exigences susmentionnées.

Hormis les poudres de PA 11, il existe toutes sortes de poudres polyamides, homopolyamides ou copolyamides, susceptibles d'être obtenues à partir de matières premières renouvelables. On peut citer par exemple les polyamides et copolyamides comprenant les monomères 10.10 ou 10.36, qui peuvent être fabriqués intégralement à partir de matières premières renouvelables, telles que les huiles végétales et les acides gras. Selon une autre alternative, les poudres polyamides peuvent être « mixtes », c'est-à-dire à base de polyamide fabriqué partiellement à partir de matières premières renouvelables. C'est le cas par exemple de l'homopolyamide 6.10 dans lequel seul l'acide sébacique (en C10) est d'origine renouvelable. C'est le cas également du copolyamide 11/10.T, dans lequel seul l'acide téréphtalique (T) n'est pas d'origine renouvelable.

Ces poudres de copolyamide sont produites par la société Arkema suivant un procédé de broyage cryo-génique. Le procédé de fabrication utilisé aujourd'hui ne permet pas d'obtenir des particules de taille moyenne inférieure à 30 $\mu$m.

La présente invention a donc pour but de concevoir une nouvelle poudre de polyamide ou de copolyamide à la fois d'origine renouvelable et de haute performance, et dont le procédé d'élaboration ne nécessite pas l'intervention de manipulations chimiques ou technologiques lourdes, coûteuses en énergie ou polluantes.

La présente invention a également pour but de fournir un procédé de fabrication de poudre de PA ultrafine qui soit simple, rapide (comportant le moins d'étapes possibles), et facile à mettre en oeuvre.

La présente invention a encore pour but de fournir un procédé modulable et flexible c'est-à-dire réglable facilement et rapidement en fonction de la granulométrie de poudre souhaitée, et qui s'adapte aisément aux dispositifs de fabrication existants dans l'industrie des poudres.

Fort de son expertise dans la fabrication de polyamide bio ressourcé haute performance, la Société déposante a maintenant trouvé une nouvelle poudre de polyamide ultrafine issue de matières premières renouvelables, et dont les domaines d'utilisation privilégiés, mais non exclusifs, sont la cosmétique, la pharmacie et la parfumerie. La demanderesse a également trouvé un procédé de fabrication de telles particules de poudre de PA de diamètre moyen inférieur à 30 $\mu$m.

Résumé de l'invention

De façon plus précise, la présente invention a donc pour objet une poudre de polyamide PA (homopolyamide ou copolyamide) issu au moins partiellement de matières renouvelables) conforme à l'objet de la revendication 1.

Avantageusement, ladite poudre de polyamide comprend au moins une des molécules suivantes : acide amino-11-undécanoïque, acide n-heptylamino-11-undécanoique, acide sébacique, décanediamine, un diacide gras, un dimère d'un acide gras et leurs mélanges.

Avantageusement, ledit polyamide PA de la poudre selon l'invention est choisi parmi le PARU, le PA10.10, les copolyamides comprenant au moins un des monomères suivants : 11, 10.10, 10.36, 6.10, 10.T, et leurs mélanges.

Avantageusement, ledit polyamide (homopolyamide ou copolyamide) est issu en totalité de matières renouvelables.

Avantageusement, la poudre de polyamide de l'invention est du PA 11.

Avantageusement, les particules de poudre de polyamide PA de l'invention ont une surface spécifique comprise dans la gamme allant de 1 à 20 m2/g, de préférence de 2 à 10 m2/g, de préférence de 3 à 6 m2/g.

Lesdites particules ont, un diamètre médian, en volume inférieur ou égal à 20 $\mu$m, de préférence compris dans la gamme allant de 5 à 15 $\mu$m.

Le pouvoir d'absorption de la poudre de l'invention, mesuré selon la norme DIN ISO 787-5, est compris dans la gamme allant de 55 à 110 g d'huile/100g de poudre, de préférence de 60 à 90 g d'huile/100g de poudre.

Avantageusement, la contrainte à la rupture de ladite poudre, lorsqu'elle est compactée uniquement par compression sous une tonne, est comprise dans la gamme allant de 100 à 600 Newtons, de préférence de 150 à 500 Newtons. Avantageusement, la poudre selon l'invention comprend du $^{14}$C. Avantageusement, ladite poudre comprend au moins 20% en masse de carbone d'origine renouvelable, de préférence 50% en masse de carbone d'origine renouvelable.

Avantageusement, ladite poudre comprend au moins 0,2.10$^{-10}$% en masse de $^{14}$C, de préférence 0,6.10$^{-10}$% en masse de $^{14}$C.

La présente invention a également pour objet l'utilisation de poudre telle que définie précédemment dans des produits cosmétiques, pharmaceutiques ou de parfumerie. Ladite poudre peut avantageusement être utilisée comme agent de compactage dans des formulations cosmétiques compactes ou bien encore comme agent matifiant.

La présente invention a également pour objet un procédé de préparation de poudre de polyamide naturel telle que définie précédemment, comprenant le broyage d'une poudre de prépolymère de viscosité inhérente inférieure à 0,5 (selon méthode ARKEMA : 0,5 g/dl dans métacrésol à 25°C), de préférence comprise dans la gamme allant de 0.25 et 0.5 (selon méthode ARKEMA : 0.5 g/dl dans métacrésol à 25°C).

Avantageusement, de la silice, de préférence pyrogénée peut être ajoutée à la poudre de prépolymère avant son broyage dans le procédé de l'invention.

Avantageusement, le broyage selon le procédé de l'invention est un broyage atmosphérique, c'est-à-dire réalisé à température ambiante.

Avantageusement, ledit broyage est effectué au moyen d'un broyeur à jets d'air opposés.

Avantageusement, ledit procédé comprend avant l'étape de broyage, une étape de fabrication d'un prépolymère de PA de masse moléculaire en nombre inférieure à 5000 g/mol, de préférence comprise dans la gamme allant de 500 à 3000 g/mol (masse moléculaire moyenne en nombre déterminée par Chromatographie d'Exclusion Stérique, en utilisant comme solvant l'HFIP, et une détection réfractométrique), et de viscosité inférieure à 0.5 (selon méthode ARKEMA : 0.5 g/dl dans métacrésol à 25°C).

Avantageusement, ledit procédé comprend après l'étape de broyage, une étape de remontée en viscosité des particules de prépolymère broyées, jusqu'à la viscosité finale désirée pour la poudre.

Avantageusement, la remontée en viscosité est effectuée par polycondensation en phase solide dans un sécheur.Avantageusement, les différentes étapes du procédé de l'invention ne font pas intervenir de solvant.

Avantageusement, la granulométrie finale de la poudre de PA est réglée directement en ajustant la vitesse de broyage. De manière avantageuse, l'ajustement de la vitesse de broyage est fait au moyen d'un sélecteur intégré au broyeur.

La présente invention a également pour objet une poudre cosmétique telle que définie précédemment, contenant un additif choisi parmi les pigments, les charges, les antioxydants et les liants de poudre.

Avantageusement, ladite poudre cosmétique contient de la poudre de silice. Avantageusement, ladite poudre cosmétique constitue un fard à joues ou à paupières.

La poudre selon l'invention peut avantageusement être utilisée dans les revêtements, les peintures, les compositions anticorrosion, les additifs pour papier, les technologies d'agglomération de poudre par fusion ou frittage provoqué par un rayonnement pour fabriquer des objets, les gels d'électrophorès, les matériaux composites multicouches, l'industrie de l'emballage, les jouets, le textile, l'automobile et/ou l'électronique.

Description détaillée

Les particules de poudre de polyamide (homopolyamide ou copolyamide) de l'invention sont issues (en totalité ou en partie seulement) de matières premières renouvelables d'origine végétale, ce qui se caractérise par le fait qu'elles comprennent du carbone d'origine renouvelable.

Par polyamide au sens de l'invention on entend les produits de condensation des lactames, des aminoacides ou des diacides avec les diamines et, en règle générale, tout polymère formé par des motifs reliés entre eux par des groupes amides.

Par polyamide d'origine totalement renouvelable entrant dans la composition de poudre selon l'invention, on entend :

- les polyamides aliphatiques obtenus à partir de lactames ou d'aminoacides (comme par exemple le PA 11 obtenu par polycondensation de l'acide amino-11-undécanoïque) ;
- les produits de condensation d'un acide dicarboxylique avec une diamine (comme par exemple le PA 10.10, produit de la condensation de la décanediamine avec l'acide sébacique, ou encore le PA 10.36, produit de la condensation de la décanediamine avec un dimère d'acide gras) ;
- les copolyamides résultant de la polymérisation de divers monomères, tels que ceux cités ci-dessus, comme par exemple les copolyamides suivants: PA 11/10.10, PA 11/10.36, PA 10.10/10.36, le copolyamide amino-11-undécanoïque/n-heptyl-11-aminoundécanoïque, etc. Les copolyamides d'origine renouvelable, qui comprennent au moins deux monomères, sont plus particulièrement décrits dans la demande de brevet française n° : 07.53319.

Le terme « monomère » dans la présente description des copolyamides doit être pris au sens d' « unité répétitive ». En effet, le cas où une unité répétitive du PA est constituée de l'association d'un diacide avec une diamine est particulier. On considère que c'est l'association d'une diamine et d'un diacide, c'est-à-dire le couple diamine.diacide (en quantité équimolaire), qui correspond au monomère. Ceci s'explique par le fait qu'individuellement, le diacide ou la diamine n'est qu'une unité structurale, qui ne suffit pas à elle seule à polymériser.

A titre d'exemples d'aminoacides d'origine renouvelable, on peut citer: l'acide 11-aminoundecanoïque produit à partir d'huile de ricin par exemple, l'acide 12-aminododecanoïque produits à partir d'huile de ricin par exemple, l'acide 10-aminodécanoïque produit à partir de l'acide decylénique obtenu par métathèse de l'acide oléïque par exemple, l'acide 9-aminononanoïque produit à partir de l'acide oléïque par exemple.

A titre d'exemples de diacides d'origine renouvelable, on peut citer, en fonction du nombre x de carbones de la molécule (Cx) :

- C4 : l'acide succinique à partir du glucose par exemple, ;
- C6 : l'acide adipique à partir du glucose par exemple ;
- C7 : l'acide heptanedioïque à partir d'huile de ricin ;
- C9 : l'acide azélaïque à partir de l'acide oléïque (ozonolyse) par exemple ;
- C10 : l'acide sébacique à partir de l'huile de ricin par exemple ;
- C11 : l'acide undécanedioïque à partir d'huile de ricin ;
- C12 : l'acide dodécanedioïque à partir de biofermentation de l'acide dodecanoïque = acide laurique (huile riche : huile de palmiste et noix de coco) par exemple ;
- C13 : acide brassylique à partir de l'acide erucique (ozonolyse) que l'on trouve dans le colza par exemple ;
- C14 : acide tetradécanedioïque par biofermentation de l'acide myristique (huile riche : huile de palmiste et noix de coco) par exemple ;
- C16 : acide hexadécanedioïque par biofermentation de l'acide palmitique (huile de palme principalement) par exemple ;
- C18 : acide octadécanedioïque obtenu par biofermentation de l'acide stéarique (un peu dans toutes les huiles végétales mais majoritaire dans les graisses animales) par exemple ;
- C20 : acide eicosanedioïque obtenu par biofermentation de l'acide arachidique (majoritaire dans l'huile de colza) par exemple ;
- C22 : acide docosanedioïque obtenu par métathèse de l'acide undécylénique (huile de ricin) par exemple
- C36 : dimère d'acide gras issu des sous-produits des résineux transfomés par les procédé Kraft.

A titre d'exemples de diamines d'origine renouvelable, on peut citer, en fonction du nombre x de carbones de la molécule (Cx) :

- C4 : butanediamine obtenu par amination de l'acide succinique ;
- C5 : pentaméthylene diamine (à partir de lysine) ; et ainsi de suite pour les diamines obtenues par amination des diacides d'origine renouvelable vus précédemment.

Par polyamide d'origine partiellement renouvelable, c'est-à-dire issu en partie seulement de matières renouvelables (nommé dans le texte polyamide « mixte »), on entend :

- les produits de condensation d'un acide dicarboxylique avec une diamine, et dans lesquels l'un des deux seulement (le diacide ou la diamine sont d'origine renouvelable. C'est le cas du PA 6.10 par exemple, puisque dans le monomère 6.10, seul l'acide sébacique est d'origine renouvelable tandis que l'héxaméthylène diamine est issue de la pétrochimie.
- les copolyamides résultant de la polymérisation de divers monomères (renouvelables, non renouvelables ou mixtes) tels que ceux cités ci-dessus. C'est le cas par exemple du CoPA 6.6/1.0.10 dans lequel le monomère « 6.6 » est d'origine non renouvelable tandis que le monomère « 10.10 » est d'origine renouvelable. C'est le cas également du PA 11/10.T par exemple, qui comporte un monomère d'origine renouvelable (« 11 ») et un monomère mixte d'origine partiellement renouvelable (« 10.T ») puisque seule la décanediamine est d'origine renouvelable, alors que l'acide téréphtalique (T) ne l'est pas.

Bien que, conformément à un mode de réalisation préféré de l'invention, la présente invention soit généralement décrite dans la suite du texte en référence à une poudre de PA 11, qui a l'avantage d'être entièrement d'origine renouvelable, la présente invention ne se limite évidemment pas aux poudres de PA 11. La présente invention inclut toute poudre de PA (homopolyamide ou copolyamide) issue totalement ou partiellement (cas des polyamides mixtes) de matières premières renouvelables, et dans laquelle les particules sont de forme non sphérique et de diamètre médian en volume inférieur à 30 μm.

Par poudre de polyamide (homopolyamide ou copolyamide) d'origine renouvelable, on entend les poudres de polyamide qui comprennent du carbone d'origine renouvelable.

En effet, à la différence des matériaux issus de matières fossiles, les matériaux composés de matières premières renouvelables contiennent du $^{14}C$. Tous les échantillons de carbone tirés d'organismes vivants (animaux ou végétaux) sont en fait un mélange de 3 isotopes : $^{12}C$ (représentant - 98,892 %), $^{13}C$ (~ 1,108 %) et $^{14}C$ (traces: $1,2.10^{-12}$ %). Le rapport $^{14}C/^{12}C$ des tissus vivants est identique à celui de l'atmosphère. Dans l'environnement, le $^{14}C$ existe sous deux formes prépondérantes : sous forme minérale c'est-à-dire de gaz carbonique ($CO2$) et sous forme organique c'est à dire de carbone intégré dans des molécules organiques.

Dans un organisme vivant, le rapport $^{14}C/^{12}C$ est maintenu constant par le métabolisme car le carbone est continuellement échangé avec l'environnement. La proportion de $^{14}C$ étant constante dans l'atmosphère, il en est de même dans l'organisme, tant qu'il est vivant, puisqu'il absorbe ce $^{14}C$ comme il absorbe le $^{12}C$. Le rapport moyen de $^{14}C/^{12}C$ est égal à $1,2\times10^{-12}$.

Le $^{12}C$ est stable, c'est-à-dire que le nombre d'atomes de $^{12}C$ dans un échantillon donné est constant au cours du temps. Le $^{14}C$, lui, est radioactif (chaque gramme de carbone d'être vivant contient suffisamment d'isotope $^{14}C$ pour donner 13,6 désintégrations par minute) et le nombre de tels atomes dans un échantillon décroît au cours du temps (t) selon la loi :

$$n = no \exp(-at)$$

dans laquelle :

- no est le nombre de $^{14}C$ à l'origine (à la mort de la créature, animal ou plante),
- n est le nombre d'atomes $^{14}C$ restant au bout du temps t,
- a est la constante de désintégration (ou constante radioactive); elle est reliée à la demi-vie.

La demi-vie (ou période), est la durée au bout de laquelle un nombre quelconque de noyaux radioactifs ou de particules instables d'une espèce donnée, est réduit de moitié par désintégration ; la demi-vie $T_{1/2}$ est reliée à la constante de désintégration a par la formule $aT_{1/2}= \ln 2$. La demi-vie du $^{14}C$ vaut 5730 ans.

Compte tenu de la demi-vie ($T_{1/2}$) du $^{14}C$, on considère que la teneur en $^{14}C$ est constante depuis l'extraction des matières premières végétales jusqu'à la fabrication du polymère, et même jusqu'à la fin de son utilisation.

Le demandeur considère qu'un polymère (ici polyamide) est issu de matières premières renouvelables s'il contient au moins 20% en masse de C d'origine renouvelable sur la masse totale de carbone, de préférence au moins 50 % en masse de C d'origine renouvelable sur la masse totale de carbone.

Autrement dit, un polymère (polyamide dans le cas de l'invention) est issu de matières premières renouvelables s'il contient au moins $0,2.10^{-10}$% en masse de $^{14}C$, de préférence $0,6.10^{-10}$% en masse de $^{14}C$.

A l'heure actuelle, il existe au moins deux techniques différentes pour la mesure de la teneur en $^{14}$C d'un échantillon :

- Par spectrométrie à scintillation liquide : Cette méthode consiste à compter des particules 'Bêta' issues de la désintégration du $^{14}$C : On mesure le rayonnement Bêta issu d'un échantillon de masse connue (nombre d'atomes $^{12}$C connu) pendant un certain temps. Cette 'radioactivité' est proportionnelle au nombre d'atomes de $^{14}$C, que l'on peut ainsi déterminer. Le $^{14}$C présent dans l'échantillon émet des rayonnements $\beta$-, qui au contact du liquide scintillant (scintillateur) donnent naissance à des photons. Ces photons ont des énergies différentes (comprises entre 0 et 156 Kev) et forment ce que l'on appelle un spectre de $^{14}$C. Selon deux variantes de cette méthode, l'analyse porte soit sur le $CO_2$ préalablement produit par l'échantillon carboné dans une solution absorbante appropriée, soit sur le benzène après conversion préalable de l'échantillon carboné en benzène.
- Par spectrométrie de masse : L'échantillon est réduit en graphite ou en $CO_2$ gazeux, analysé dans un spectromètre de masse. Cette technique utilise un accélérateur et un spectromètre de masse pour séparer les ions $^{14}$C des $^{12}$C et donc déterminer le rapport des deux isotopes.

Toutes ces méthodes de mesure de la teneur en $^{14}$C des matériaux sont décrites précisément dans les normes ASTM D 6866 (notamment D6866-06) et dans les normes ASTMD 7026 (notamment 7026-04). Ces méthodes mesurent le rapport $^{14}$C/$^{12}$C d'un échantillon et le comparent avec le rapport $^{14}$C/$^{12}$C d'un échantillon référence d'origine 100% renouvelable, pour donner un pourcentage relatif de C d'origine renouvelable dans l'échantillon.

La méthode de mesure préférentiellement utilisée dans le cas des polyamides de l'invention est la spectrométrie de masse décrite dans la norme ASTM D6866-06 (« accelerator mass spectroscopy »).

Les particules de poudre de polyamide selon l'Invention, telles que le PA11, sont de forme irrégulière non sphérique. Lesdites particules ne présentent pas d'arrêtés vives, ce qui leur procure un effet glissant et roulant au toucher.

Le diamètre moyen (diamètre médian en volume) des particules de polyamide de l'invention est inférieur ou égal à 20 $\mu$m, et de préférence compris dans la gamme allant de 5 à 15 $\mu$m, ou mieux sensiblement égal à 10 $\mu$m.

Avantageusement, la surface spécifique des particules de poudre selon l'invention est comprise dans la gamme allant de 1 à 20 m$^2$/g, de préférence de 2 à 10 m$^2$/g, de préférence de 3 à 6 m$^2$/g. Le pouvoir d'absorption desdites particules, mesuré selon la norme DIN ISO 787-5 est compris dans la gamme allant de 55 à 110 g d'huile/100g de poudre, de préférence de 60 à 90 g d'huile/100g de poudre de PA.

La poudre de l'invention présente des propriétés de contrôle du sébum, ainsi qu'un effet matifiant. Elle est donc parfaitement bien adaptée à des produits cosmétiques destinés au maquillage et/ou au soin de la peau humaine, en particulier du visage, du cou et du corps, ainsi qu'aux produits pharmaceutiques ou de parfumerie (poudre parfumante pour le corps ou les pieds par exemple).

De manière avantageuse, les poudres de l'invention offrent, de par leur forme, leur granulométrie et leur surface spécifique, des propriétés sensorielles améliorées, ainsi que des propriétés de compactage et des propriétés d'absorption d'huile améliorées, comparativement aux poudres de polyamide de l'art antérieur.

Des poudres de diamètre sensiblement égal à 10 $\mu$m de PA 12 (origine non renouvelable) et de PA 11 (origine renouvelable) ont été évaluées sous forme libre par un panel sensoriel entraîné composé de 10 personnes. Les descripteurs utilisés sont listés ci-après. Ils sont évalués sur une échelle allant de 1 à 10. Sur cette échelle, « 1 » représente des propriétés inférieures d'étalement, de pouvoir couvrant ou de velouté, tandis que « 10 » représente des propriétés supérieures d'étalement, de pouvoir couvrant ou de velouté.

<u>FACILITE D'ETALEMENT :</u>

Définition : Caractérise la facilité d'étaler la poudre.
Protocole : Prendre une pointe de spatule de poudre, la poser sur la tabatière et l'étaler en couche mince
Evaluation : Noter la facilité à étaler la poudre (la sensation glissante).

<u>POURVOIR COUVRANT :</u>

Définition : Caractérise la propriété de la poudre à recouvrir la peau de façon uniforme, blanchissant.
Protocole : Prendre une pointe de spatule de poudre, la poser sur la tabatière et exercer un balayage avec la main (étaler la poudre avec la main en un aller-retour).
Evaluation : Noter l'homogénéité de l'étalement, l'uniformité de la couche, visuellement.

VELOUTE:

Définition : Caractérise une première sensation glissante (velouté, onctueux) qui reste sans altération dans la durée.
Protocole : Prendre une pincée entre les doigts et effectuer des petits mouvements circulaires sans pression.
Evaluation : Noter la sensation de douceur sur la durée.

**Tableau 1: Résultats (moyenne des notes) des tests sensoriels effectués sur PA 11 et PA 12 broyés**

|  | Etalement | Couvrance | Velouté |
|---|---|---|---|
| **Polyamide 11 broyé** | 8 | 4,3 | 3 |
| **Polyamide 12 broyé** | 4,5 | 6 | 3,2 |

Les résultats de l'analyse sensorielle montrent que la poudre de polyamide 11 obtenue selon le procédé de l'invention présente le même niveau de velouté que la poudre de polyamide 12 obtenue par broyage selon le procédé décrit dans la demande de brevet DE 4421454. Le polyamide 11 et le polyamide 12 présentent des propriétés sensorielles analogues.

De plus, la poudre de polyamide 11 broyé présente un pouvoir d'étalement supérieur à celui de la poudre de Polyamide 12, corrélé à un pouvoir couvrant inférieur qui apporte un fini naturel. Cette caractéristique est liée à la répartition granulométrique, et peut être aisément ajustée en fonction des conditions de broyage, grâce au procédé de l'invention.

De plus, la poudre PA de l'invention, notamment grâce à la forme non sphérique de ses particules, présente des propriétés de compactage améliorées, par rapport aux autres poudres polyamides obtenues selon les procédés de l'art antérieur (et qui sont de forme sphérique), ce qui permet de l'utiliser avantageusement comme poudre compactée de maquillage, notamment comme fard à joues ou à paupières.

Ainsi, la contrainte à la rupture de ladite poudre, lorsqu'elle est compactée uniquement par compression sous une tonne, est comprise dans la gamme allant de 100 à 600 Newtons, de préférence de 150 à 500 Newtons.

Le pouvoir de compaction est évalué de la façon suivante :

1- Réalisation du compact

Chaque compact est réalisé avec $0.5 \pm 0,002$g de poudre pour faire une pastille de 13mm de diamètre. La compression est réalisée en trois phases :

1ère compression à 1 tonne : lors de cette première phase, la pression sur le compact chute très vite (réduction de l'air et empilement optimal de la poudre). Après 5 secondes la pression est relâchée.
2ème compression à 1 tonne : la poudre se compacte et la pression retombe très peu. Après 5 secondes la pression est relâchée.
3ème compression à 1 tonne de la poudre pendant 5 secondes.

Après ces 3 séries de compression la poudre est compactée sous forme d'un comprimé de 13mm de diamètre et 4,5mm d'épaisseur.

2- Mesure de la cohésion des compacts

L'essai mécanique pour tester ces compacts est très fréquemment utilisé en pharmacie pour caractériser les comprimés (Test de rupture diamétrale ou test « brésilien »). Ce test consiste à appliquer une force perpendiculairement à la direction de compression (c'est-à-dire sur la tranche du compact) jusqu'à la rupture du comprimé.

Le tableau 2 suivant donne les résultats de contrainte à la rupture pour différents types de poudre de polyamide.

**Tableau 2 : Résultats des tests de rupture pour différents types de poudre PA**

|  | Contrainte à la rupture (Newtons) | Remarque |
|---|---|---|
| Poudre PA11 broyée (10 $\mu$m) | 160 |  |

(suite)

|  | Contrainte à la rupture (Newtons) | Remarque |
|---|---|---|
| Poudre PA11 broyée (100 um) | 5 | |
| Poudre sphérique de PA 12 (10 μm) | 0 | Pas de compaction |
| Poudre sphéroïdale poreuse de PA12 (10 μm) | 240 | |

Le tableau 2 montre qu'une poudre sphérique de PA12 ne peut pas être compactée. Il en serait de même pour une poudre sphérique de PA11. En revanche, la poudre de PA11 obtenue par le nouveau procédé de broyage se compacte très bien, puisqu'on obtient un comprimé qui se tient mécaniquement uniquement par compression, sans ajout de composé gras. La résistance mécanique du comprimé obtenue est légèrement inférieure à celle obtenue avec la poudre sphéroïdale poreuse de Polyamide 12 mise au point spécifiquement pour cette propriété de compaction. La poudre de PA 11 selon l'invention peut donc être avantageusement utilisée comme agent de compactage, particulièrement bien adapté aux formulations cosmétiques de poudres compactes.

Par ailleurs, la poudre de PA11 de l'invention présente des propriétés intéressantes en émulsion : elle diminue l'aspect collant et gras des émulsions à phase grasse continue. Elle laisse sur la peau un fini mat et poudré qui change totalement le confort d'utilisation des formulations incluant la poudre de l'invention.

En effet, les compositions cosmétiques à phase grasse continue provoquent très souvent des désagréments à l'application qui limitent parfois leur utilisation par les consommateurs. Le film gras continu à la surface de la peau provoque une sensation de collant, d'huileux et de poisseux, qui n'est plus acceptée par les consommateurs d'aujourd'hui. Par ailleurs, l'aspect brillant et huileux nuit aux propriétés cosmétiques et esthétiques de ces crèmes. L'application d'un maquillage après l'application de crème de jour ou solaire est rendue difficile à cause de cet effet collant qui interfère dans l'étalement du maquillage sur la peau. L'application du maquillage est donc perturbée, provoquant des inhomogénéités. Au cours du temps, on assiste également à une mauvaise tenue du maquillage ainsi qu'à un transfert et à une perte des couleurs du maquillage.

Pour réduire l'aspect huileux, collant et poisseux de ces compositions à phase grasse continue, il est connu d'ajouter des huiles volatiles telles que les huiles de silicone mais elles ne jouent pas le rôle protecteur associé à une phase grasse continue et n'ont pas de propriétés humectantes de la peau. Par ailleurs, les polyols ajoutés généralement dans les formulations pour leurs propriétés humectantes et hydratantes de la peau apportent un effet poisseux persistant, indésirable sur la peau, qui s'ajoute à celui des huiles cosmétiques de la composition.

Il est donc important de fabriquer une composition cosmétique répondant à la fois au problème sensoriel et esthétique tout en remplissant son rôle protecteur vis-à-vis de la peau tel qu'évoqué plus haut. Une émulsion eau/huile comprenant une poudre fine selon l'invention permet de résoudre ce problème technique. Elle réduit notamment de façon significative l'effet gras et poisseux apporté par les huiles et/ou les polyols des compositions cosmétiques. Cet effet est observé quel que soit le type d'huile, qu'elle soit volatile ou pas, qu'elle soit d'origine minérale, animale, végétale ou de synthèse, et qu'elle soit hydrocarbonée, siliconée ou fluorée.

A titre d'exemple, on peut citer la formulation suivante (nommée ci-après « composition I ») d'une émulsion Eau/Silicone et contenant de la glycérine :

| Phase | Ingrédients (noms INCI) | % poids |
|---|---|---|
| A | Eau | QSP100 |
| | Magnésium sulfate | 0.70 |
| | Disodium EDTA | 0.10 |
| | Glycerin | 3.00 |
| | Chlorophenesin | 0.25 |
| | Phenoxyethanol, Methylparoben, Ethylparaben, | 0.60 |
| | Butylparaben, Propylparaben, Isobutylparaben Xanthan Gum | 0.10 |
| B | Cyclopentasiloxane, PEG-10 Dimethicone, Disteardimonium Hectorite | 8.00 |
| | Cyclomethicone | 20.00 |
| | Disteardimonium Hectorite | 1.00 |
| C | Poudre de Polyamide 11 | 3.5 |
| Procédé de fabrication : | | |

(suite)

| Phase | Ingrédients (noms INCI) | % poids |
|---|---|---|
| Mélanger les ingrédients de la phase A. | | |
| Mélanger les ingrédeients de la phase B. | | |
| Ajouter A dans B lentement en agitant à haute vitesse pour faire l'émulsion. | | |
| Ajouter la poudre de polyamide 11 dans l'émulsion sous agitation douce. | | |

L'effet de l'addition de poudres PA selon l'invention dans des émulsions à phase grasse continue a été mesuré par analyse sensorielle dans différents types de compositions. Chaque composition a fait l'objet d'une étude de profil sensoriel, conduite par un panel de cinq experts selon les descriptifs suivants :

- pendant la phase d'application du produit (le gras, la rapidité de pénétration) ; et
- immédiatement après application (le brillant de la peau, la douceur de la peau, l'effet peau collante).

Chaque composition est analysée en aveugle par comparaison de tous les essais formant une série. Les différents critères ont été évalués sur une échelle allant de 0 à 8. La valeur 0 indiquant l'absence du critère désigné (par exemple une sensation d'absence de gras) ; la valeur 8 indiquant une tendance très marquée pour le critère choisi (par exemple une sensation très importante de présence de gras).

Les résultats sont rassemblés dans le tableau 3, montrant le comportement lors de l'application d'une émulsion à phase grasse continue incluant la poudre de l'invention (Composition I) en comparaison avec la même émulsion ne contenant pas la poudre de l'invention (Témoin).

La Composition I comprend 3,5% de poudre de Polyamide 11 (correspondant à l'exemple 7 décrit dans la suite du texte), le broyage ayant été suivi d'une remontée en viscosité pour atteindre une viscosité en solution de 0,8 (selon méthode ARKEMA : 0.5g/ dl dans métacrésol à 25°C).

**Tableau 3**

| | | Témoin 0% de PA 11 | **Composition I** 3,5% de PA 11 |
|---|---|---|---|
| Comportement lors de l'application | Gras | 6 | 2 |
| | Rapidité de pénétration | 1 | 5 |
| Comportement après l'application | Brillant | 8 | 4 |
| | Douceur | 2 | 4 |
| | Peau collante | 6 | 2 |

Les caractéristiques intrinsèques de la poudre PA 11 selon l'invention confèrent aux formulations qui en contiennent en quantité efficace, un toucher doux lors de la prise, une absorption très rapide pendant l'application sur la peau (par exemple en 2 ou 3 gestes seulement), et un fini mat sur la peau. Comparativement aux autres poudres matifiantes de l'art antérieur, les poudres PA 11 apportent un effet velours ou « velouté » plus léger et un fini naturel et imperceptible. Le maquillage naturel est de plus en plus recherché par les consommateurs.

La poudre non sphérique de la présente invention est donc particulièrement bien adaptée aux produits cosmétiques, pour hommes notamment. Ladite poudre permet de créer des formulations pour lesquelles à la fois le geste d'application et le fini sur la peau sont spécifiquement masculins.

Bien qu'elles soient destinées au domaine cosmétique (pour femme et /ou pour homme) dans les modes de réalisation décrits, les poudres de l'invention peuvent être utilisées dans tous les autres domaines où leurs propriétés, notamment de granulométrie, de compaction, et leur origine renouvelable sont recherchées.

A titre d'exemples, les poudres de l'invention sont particulièrement bien adaptées aux revêtements (anticorrosion, peintures, etc). Les poudres de l'invention peuvent également être utilisées comme additifs pour papier ou bien encore en électrophorèse ou dans les technologies d'agglomération de poudre par fusion ou frittage provoqué par un rayonnement, tel que par exemple un faisceau laser (laser sintering) pour fabriquer des objets. Lesdites poudres peuvent en outre être utilisées en tant qu'espaceurs dans les matériaux composites, notamment entre les couches de matériaux multicouches. Leurs utilisations dans l'industrie de l'emballage, des jouets, du textile, l'automobile et l'électronique sont également envisageable.

La présente invention a également pour objet un procédé de préparation d'une poudre de polyamide telle que définie ci-dessus. Le procédé se caractérise en ce qu'il comprend le broyage d'une poudre de prépolymère de faible viscosité inhérente.

Si le procédé de l'invention est dans la présente description, conformément à un mode de réalisation préféré (mais non limitatif) de l'invention, appliqué à la fabrication de poudre de polyamide 11, il va de soi que les mêmes étapes de ce procédé sont transposables dans le cas de la fabrication d'autres types d'homopolyamides ou copolyamides.

De manière connue, le polyamide 11 est obtenu par polycondensation de l'acide amino-11 undécanoïque ou lactame 11.

Selon un mode de réalisation du procédé de l'invention, le prépolymère utilisé est un polyamide 11 de masse moléculaire moyenne en nombre basse et de faible viscosité.

De la même façon, dans le cas de la fabrication d'une autre poudre de copolyamide, le procédé de l'invention utilise un prépolymère de CoPA de faible masse moléculaire moyenne en nombre et de faible viscosité. De manière avantageuse, un tel prépolymère de CoPA comprend au moins deux monomères, dont l'un est majoritaire (c'est à dire dont le pourcentage en poids sur le poids total de CoPA est supérieur à 50%). Le monomère majoritaire est avantageusement choisi de sorte que la température de fusion du CoPA ne soit pas trop basse, mais plutôt de l'ordre de 170°C par exemple, de sorte qu'il n'est pas nécessaire de faire le broyage de l'invention dans des conditions cryogéniques. De préférence, le monomère majoritaire d'un tel CoPA est l'acide amino-11-undécanoïque.

Ce prépolymère de faible viscosité présente l'avantage d'être très facilement broyable, et donc de permettre son broyage dans des conditions moins sévères que dans les procédés classiques de broyage de polyamide.

Le prépolymère (PA 11 ou autres) de l'invention a une masse moléculaire en nombre (Mn) mesurée par GPC inférieure à 5000 g/mol, de préférence comprise dans la gamme allant de 500 et 3000 g/mol.

Par faible viscosité, on entend une viscosité inhérente du prépolymère inférieure à 0,5 (mesure pour 0,5 g/dl dans le métacrésol à 25°C).

La viscosité inhérente du prépolymère est avantageusement comprise dans la gamme allant de 0,25 à 0,50, de préférence dans la gamme allant de 0.30 à 0.45. Bien entendu, la viscosité inhérente du polyamide peut être plus faible encore si nécessaire, en fonction de l'application visée. En effet, selon le procédé de l'invention, plus le prépolymère de départ est de faible viscosité, plus il est facile à broyer et plus le diamètre moyen de la poudre de PA obtenue sera petit. Grâce à la flexibilité ou modularité du procédé de l'invention, il suffit donc d'adapter au départ la viscosité du prépolymère en fonction de la granulométrie visée pour la poudre.

Pour obtenir un prépolymère de faible viscosité, de l'amino-11 est par exemple chargé dans un autoclave avec 30 à 50% d'eau, avec ajout optionnel d'un catalyseur, tel que l'acide phosphorique. L'ajout d'un catalyseur dans le procédé de l'invention n'est généralement pas nécessaire, et il est possible de partir d'un système sans aditif phosphoré. Le mélange est chauffé à une température de 790°C environ, sous une pression de 10 bars. L'eau est distillée et le réacteur est dégazé. La vapeur prélevée est recondensée et pesée. Un suivi de la quantité de vapeur retirée est effectué jusqu'à une certaine quantité de vapeur retirée, qui correspond à la viscosité souhaitée pour le prépolymère. On vidange alors le prépolymère de viscosité adéquate. Au niveau de la vanne de vidange, le prépolymère est encore en fusion puis il refroidit sous l'effet du départ de la vapeur de l'eau et se retrouve solidifié. Le prépolymère solidifié est ensuite passé dans un granulateur ou un broyeur, qui le réduit en poudre grossière de diamètre moyen inférieur à 3 mm

A ce stade, le prépolymère obtenu peut avantageusement subir un compoundage de façon à lui ajouter toutes sortes d'additifs, tels que des pigments, des antioxydants, des liants de poudre, etc. Des poudres de toutes les couleurs peuvent ainsi être fabriquées. Le compoundage consiste à effectuer le mélange en phase fondue du PA avec des additifs (par exemple au moyen de deux vis dans un fourreau chauffé). Le mélange est ensuite refroidi à l'aide de deux rouleaux en acier avec circulation d'eau froide ou bien à l'aide d'une calandreuse. Le prépolymère est ensuite réduit en granules et peut subir un broyage grossier, avant d'alimenter le broyeur. L'alimentation en PA du broyeur est réalisée en continu par un système de sas à double vanne.

Le broyage selon l'invention est un broyage atmosphérique, c'est-à-dire réalisé à température ambiante (25°C environ). Un tel broyage permet d'éviter la formation d'arrêtes vives à la surface des particules de poudre, ce qui a une influence importante sur les propriétés sensorielles, en particulier de toucher de la poudre obtenue.

De plus, l'impact environnemental du procédé de l'invention est beaucoup plus faible que celui d'un procédé de dissolution/précipitation en milieu solvant qui demande de chauffer, puis de refroidir des quantités importantes de solvants généralement issus de la pétrochimie. Ces derniers devront être finalement éliminés, même si un circuit de recyclage permet de les réutiliser plusieurs fois.

Le dispositif de broyage utilisé dans le procédé de l'invention peut être de tout type approprié pour la fabrication de poudres.

Selon un mode de réalisation préféré de l'invention, le broyage est effectué au moyen d'un broyeur à jets d'air opposés, grâce à deux buses opposées alimentées par de l'air comprimé, généralement à 8,5 bar ($8,5 \times 10^5$ Pa). L'air utilisé de préférence est filtré et séché, donc il n'apporte pas de contaminant. Bien entendu, tout autre gaz approprié pourrait remplacer l'air alimentant les buses. Le prépolymère est véhiculé et emporté directement par l'air qui sort des buses. Sous l'effet des jets d'air opposés, les particules de prépolymère entrent en collision l'une contre l'autre, ce qui réduit leur granulométrie et conduit à leur forme finale caractéristique irrégulière et sans arête vive.

Les dimensions du système de broyage et les débits d'entrée de gaz utilisés sont adaptés pour obtenir une bonne fluidisation et la granulométrie désirée. A titre d'exemple, la puissance consommée pour le broyage est d'environ 1 à 2 kW.h/kg de poudre.

Ce type de broyeur est couramment utilisé et largement répandu dans l'industrie des polymères. Le broyeur à jets d'air opposés est particulièrement bien adapté pour la fabrication de poudres très fines et présentant des courbes de distribution de granulométrie étroites. En effet, comparés à la vitesse relative des broyeurs mécaniques (broyeurs à impact: jusqu'à 140 m/s, broyeurs contra-rotatifs: jusqu'à 250 m/s), les broyeurs à jets d'air opposés utilisés dans le procédé de l'invention permettent des vitesses relatives de broyage beaucoup plus élevées, supérieures à 400 m/s.

De manière avantageuse, le broyeur à jets d'air opposés comporte un classificateur ou sélecteur intégré, capable de régler directement la vitesse de broyage pour obtenir la granulométrie désirée, à la différence d'autres systèmes qui nécessitent généralement de prévoir l'adjonction en série d'un dispositif de réglage annexe. Le sélecteur renvoie les particules de diamètre non conforme au système d'alimentation de la chambre de broyage tandis que les particules de granulométrie conforme au réglage sont collectées dans un filtre à air. La poudre peut être collectée directement au pied de ce filtre, en sac par exemple. Les seuls réglages à faire selon le procédé de l'invention sont la vitesse de broyage pour obtenir la granulométrie désirée, et le débit d'alimentation pour maintenir une certaine quantité de produit constante dans la chambre de broyage. La vitesse de broyage peut être réglée directement au niveau du sélecteur et les transitions pour changer la granulométrie de la poudre au cours du procédé de l'invention sont extrêmement rapides. L'utilisation d'un tel broyeur à jets d'air opposés améliore la productivité du procédé de l'invention.

Les exemples suivants illustrent un mode de réalisation préféré de l'invention, sans toutefois la limiter. Le broyeur utilisé dans ces exemples est un broyeur à jets d'air opposés (2 buses). Il s'agit d'un gros broyeur de débit d'air sensiblement égal à 1250 m3/h (modèle: MultiNO 6240, fabricant : Schüttgutveredelung NOLL GmbH).

Exemple 1 :

Fabrication de poudre de D50 = 8μm., le D50 correspondant à la taille moyenne en volume, c'est à dire la valeur de la taille de particule qui divise la population de particules examinée exactement en deux.

En quelques minutes à peine, l'homme du métier trouve le réglage de la vitesse du sélecteur pour obtenir un D50 de 81im. 4 sacs de 15 kg de poudre (soit 60 kg au total) sont produits avec un débit de 60 kg/h (donc une énergie consommée de 2 kW.h/kg de poudre). La vitesse du sélecteur est de 1900 tr/mn. Le D50 est très stable durant la production :

- sac N° 1 : D50 = 8.06 μm
- sac N° 2 : D50 = 8.07 μm
- sac N° 3 : D50 = 8.11 μm
- sac N° 4 : D50 = 8.10 μm

Exemple 2 :

Fabrication de poudre D50 = 12μm :
Débit de 150 kg/h avec vitesse sélecteur 1150 tr/mn. 4 sacs de 15 kg sont fabriqués soit 60 kg.

Exemple 3 :

Fabrication de poudre D50 = 10μm :
Débit de 100 kg/h avec vitesse sélecteur de 1450 tr/mn. D50 stables également :

- sac N° 1 : D50 = 10.33 μm
- sac N° 2 : D50 = 10.15 μm
- sac N° 3 : D50 = 10.08 μm
- sac N° 4 : D50 = 10.33 μm
- etc.

Par ailleurs, puisque le broyeur à jets d'air ne comporte pas d'organes de broyage, son nettoyage et sa maintenance en sont facilités, et il ne présente aucun des problèmes d'usure ou de risques de contamination de la poudre pouvant être rencontrés avec les organes de broyage classique. Le broyeur à jets d'air permet en outre d'éviter le risque de pollution de la poudre par des particules métalliques, comparativement aux broyeurs qui utilisent des pièces métalliques en mouvement. La pureté, et la qualité naturelle et renouvelable des poudres de polyamide selon l'invention sont donc préservées grâce à ce mode de réalisation du procédé de l'invention.

Bien entendu, le dispositif de broyage utilisé par l'invention n'est pas limité à ce mode de réalisation et tout autre dispositif approprié, tel qu'un broyeur à boulets, broyeur à billes, broyeur à rouleaux, etc, pourrait aussi être utilisé, à condition de permettre l'obtention d'une poudre conforme à celle définie par la présente invention.

Le dispositif de broyage du procédé de l'invention permet d'obtenir des particules de poudre de diamètre moyen inférieur à 30 $\mu$m, et de préférence inférieur ou égal à 20 $\mu$m. Le diamètre moyen peut avantageusement être inférieur à 5 $\mu$m grâce au procédé de l'invention. Le diamètre moyen des particules de poudre obtenues par le procédé de l'invention est choisi en fonction de l'application visée. Par exemple pour la cosmétique, le diamètre moyen des particules de poudre est avantageusement compris dans la gamme allant de 5 et 15 $\mu$m, et de préférence il est sensiblement égal à 10 $\mu$m.

Selon un mode de réalisation du procédé de l'invention, et pour améliorer encore la « processabilité » sur les grades ultra-fins, on peut ajouter à la poudre de prépolymère, préalablement à son broyage, 1% en masse de silice, de préférence pyrogénée, par rapport au poids total (PA - silice). On peut citer par exemple les produits commercialisés sous les dénominations AEROSIL (tel que l'Aérosil R972) par la société DEGUSSA Evonik, ainsi que les produits commercialisés sous les dénominations CAB-O-SIL par la société CABOT.

Selon un mode de réalisation préféré de l'invention, la poudre obtenue après broyage est soumise à une polycondensation en phase solide dans un sécheur. Cette étape aussi appelée « séchage » sert à effectuer une remontée en viscosité des particules de prépolymère broyées. Lors de cette étape de remontée en viscosité, la poudre est par exemple chauffée (à une température d'environ 150°C) sous vide sous une pression d'environ 20 mbar absolus (soit $2 \times 10^3$ Pa) dans un sécheur rotatif pendant un temps variable, dépendant de la viscosité finale de poudre souhaitée. D'autres techniques appropriées de remontée en viscosité peuvent bien entendu être employées dans le procédé de l'invention, telles que le chauffage sous balayage d'azote, le séchage au moyen de bi-cônes rotatifs ou encore au moyen de bi-cônes agités, etc.

Selon un mode de réalisation préféré de l'invention, un tamisage de sécurité (ou « screening ») est en outre effectué pour enlever les agglomérats ou autres grosses particules qui peuvent s'être créées au cours du séchage.

La poudre de PA (homopolyamide ou copolyamide) obtenue est alors prête pour être conditionnée, en sacs par exemple.

La présente invention fournit donc un procédé d'élaboration de poudre (notamment cosmétique) d'origine renouvelable et de haute performance, dans lequel le produit initial (prépolymère) subit peu de transformations chimiques : principalement des transformations mécaniques primaires, telles que le broyage ou le filtrage mécanique. Le procédé de l'invention permet d'éviter les deux étapes de dissolution et précipitation et ne nécessite pas l'utilisation de solvants. Il est donc respectueux de l'environnement. De plus le procédé d'élaboration de poudre selon l'invention laisse peu de résidus, ceux-ci étant aisément recyclables.

En outre, par rapport aux produits naturels (poudres de noyaux de fruit, poudre de riz, de bambou, etc...), les poudres de l'invention présentent les avantages de matériaux de synthèse. Leurs caractéristiques (notamment leurs impuretés) sont parfaitement contrôlées par les conditions de synthèse. Le procédé de l'invention limite les risques de contamination par des composés toxiques ou allergènes. Les poudres ainsi obtenues ne sont pas contaminées sur le plan bactériologique ou mycologique et ne nécessitent pas d'étape ultérieure de décontamination ni d'ajout de conservateurs.

Les exemples 4 à 7 illustrent différents modes de réalisation du procédé selon l'invention sans toutefois la limiter, et indiquent les caractéristiques de granulométrie de la poudre obtenue sur granulomètre Cilas 920, fonctionnant par diffraction laser. Le broyeur utilisé dans ces exemples est un broyeur à jets d'air opposés (2 buses). Il s'agit d'un petit broyeur pilote, de débit d'air sensiblement égal 100 m3/h (modèle: MultiNO 1290, fabricant : Schüttgutveredelung NOLL GmbH).

Exemple 4 :

Un prépolymère sous forme de chips de polyamide 11 (produit A), de viscosité relative 0,45 (selon méthode ARKEMA : 0,5 g/dl dans métacrésol à 25°C), est chargé dans un broyeur bicône à boulets en acier discontinu. Après une durée de rotation de plusieurs heures, la poudre est vidangée. la granulométrie obtenue mesurée sur granulomètre Cilas 920 est :

D50 = 15,6$\mu$m D10 = 7,6$\mu$m D90 = 26,2$\mu$m

Exemple 5 :

Le produit A (Cf. exemple 4) est préalablement broyé dans un boyeur/classificateur à un diamètre moyen proche de 90μm, puis additionné de 1 % en Aérosil 972 (fournisseur Degussa AG), ce pourcentage étant donné en masse par rapport au poids total PA 11 + silice. Cette poudre est alors alimentée dans un broyeur à jets d'air opposés. Le débit d'air comprimé à 5 bar effectifs est de 100 Nm$^3$/h et la vitesse de rotation du classificateur est de 9000 rpm. La granulométrie de la poudre obtenue mesurée sur granulomètre Cilas 920 est :
D50 = 10μm D10 = 1,6μm D90 = 19,6μm

Exemple 6 :

Un prépolymère sous forme de chips de polyamide 11 (produit B), de viscosité relative 0,3 (selon méthode ARKEMA : 0,5 g/dl dans métacrésol à 25°C) et additionné de 1% en masse d'Aérosil 972, est alimentée dans un broyeur à jets d'air opposés. Le débit d'air comprimé à 5 bar (5x10$^5$ Pa) effectifs est de 100 Nm$^3$/h et la vitesse de rotation du classificateur est de 9000 rpm (tour par minute). La granulométrie de la poudre obtenue mesurée sur granulomètre Cilas 920 est :
D50=5,1μm D10=1,2μm D90=11,5μm

Exemple 7 :

Un prépolymère sous forme de chips de polyamide 11 (produit B), de viscosité relative 0,3 (selon méthode ARKEMA : 0,5 g/dl dans métacrésol à 25°C) et additionné de 1% en masse en Aérosil 972, est alimentée dans un broyeur à jet d'air opposés. Le débit d'air comprimé à 5 bar (5x10$^5$ Pa) effectifs est de 100 Nm$^3$/h et la vitesse de rotation du classificateur est de 6000 rpm (tour par minute). La granulométrie de la poudre obtenue mesurée sur granulomètre Cilas 920 est :
D50 = 8,9μm D10 = 1,5μm D90 = 18,4μm

En définitive, le procédé de fabrication de poudre polyamide ultrafine de l'invention permet de s'affranchir de la consommation de pétrole, de réduire la consommation d'énergie, et de faire appel à des matières premières issues de la culture de végétaux. Il présente, en outre, un coût plus faible de fabrication et un bilan énergétique favorable.

**Revendications**

1.  Poudre de polyamide PA (homopolyamide ou copolyamide) issu au moins partiellement de matières renouvelables, dans laquelle les particules sont de forme non sphérique, irrégulière, sans arête vive et de diamètre médian en volume compris dans la gamme allant de 5 à 20 μm mesuré par un granulomètre Cilas 920 par diffraction laser, ladite poudre présentant un pouvoir d'absorption mesuré selon la norme DIN ISO 787-5 compris dans la gamme allant de 55 à 110 g d'huile/100g de poudre de polyamide, et présentant une contrainte à la rupture comprise dans la gamme allant de 100 à 600 Newtons, lorsque la poudre est compactée uniquement par compression sous une tonne.

2.  Poudre de polyamide selon la revendication 1, **caractérisée en ce qu'**elle comprend au moins une des molécules suivantes : acide amino-11-undécanoïque, acide n-heptylamino-11-undécanoïque, acide sébacique, décanediamine, un diacide gras, un dimère d'un acide gras et leurs mélanges.

3.  Poudre de polyamide selon la revendication 1 ou 2, **caractérisée en ce que** le PA est choisi parmi le PA11, le PA10.10, les copolyamides comprenant au moins un des monomères suivants : 11, 10.10, 10.36, 6.10, 10.T, et leurs mélanges.

4.  Poudre de polyamide selon l'une quelconque des revendications précédentes, **caractérisée en ce que** ledit polyamide (homopolyamide ou copolyamide) est issu en totalité de matières renouvelables.

5.  Poudre selon l'une quelconque des revendications précédentes, dans laquelle les particules ont une surface spécifique comprise dans la gamme allant de 1 à 20 m$^2$/g, de préférence de 2 à 10 m$^2$/g, de préférence de 3 à 6 m$^2$/g.

6.  Poudre selon l'une quelconque des revendications précédentes, dans laquelle les particules ont un diamètre médian en volume compris dans la gamme allant de 5 à 15 μm.

7. Poudre selon l'une quelconque des revendications précédentes, **caractérisée en ce que** son pouvoir d'absorption mesuré selon la norme DIN ISO 787-5 est compris dans la gamme allant de 60 à 90 g d'huile/100g de poudre de polyamide.

8. Poudre selon l'une quelconque des revendications précédentes, **caractérisée en ce que** la contrainte à la rupture de ladite poudre, lorsqu'elle est compactée uniquement par compression sous une tonne, est comprise dans la gamme allant de 150 à 500 Newtons.

9. Poudre selon l'une quelconque des revendications 1 à 8, **caractérisée en ce qu'**elle comprend du $^{14}$C.

10. Poudre selon l'une quelconque des revendications 1 à 9, comprenant au moins 20% en masse de carbone d'origine renouvelable.

11. Poudre selon l'une quelconque des revendications 1 à 10, comprenant au moins $0,2.10^{-10}$ % en masse de $^{14}$C.

12. Utilisation de poudre selon l'une quelconque des revendications précédentes dans des produits cosmétiques, pharmaceutiques ou de parfumerie.

13. Utilisation de poudre selon la revendication 12 comme agent de compactage dans des formulations cosmétiques compactes.

14. Utilisation de poudre selon la revendication 12 ou 13, comme agent matifiant.

15. Procédé de préparation de poudre de polyamide selon l'une quelconque des revendications 1 à 11, comprenant :

   - le broyage à température ambiante d'une poudre de prépolymère de viscosité inhérente inférieure à 0,5 (selon méthode ARKEMA : 0,5 g/dl dans métacrésol à 25°C), ledit broyage étant effectué au moyen d'un broyeur à jets d'air opposés.

16. Procédé selon la revendication 15, dans lequel le prépolymère à une viscosité inhérente comprise dans la gamme allant de 0.25 à 0.5 (selon méthode ARKEMA : 0.5 g/dl dans métacrésol à 25°C).

17. Procédé selon la revendication 15 ou 16, dans lequel de la silice pyrogénée est ajoutée à la poudre de prépolymère avant son broyage.

18. Procédé selon l'une quelconque des revendications 15 à 17, comprenant avant l'étape de broyage, une étape de :

   - fabrication d'un prépolymère de PA de masse moléculaire en nombre inférieure à 5000 g/mol, et de viscosité inférieure à 0.5 (selon méthode ARKEMA : 0.5 g/dl dans métacrésol à 25°C).

19. Procédé selon l'une quelconque des revendications 15 à 18, comprenant après l'étape de broyage, une étape de :

   - remontée en viscosité des particules de prépolymère broyées, jusqu'à la viscosité finale désirée pour la poudre.

20. Procédé selon l'une quelconque des revendications 15 à 19, dans lequel la remontée en viscosité est effectuée par polycondensation en phase solide dans un sécheur.

21. Procédé selon l'une quelconque des revendications 15 à 20, **caractérisé en ce que** ses différentes étapes ne font pas intervenir de solvant.

22. Procédé selon l'une quelconque des revendications 15 à 21, dans lequel la granulométrie finale de la poudre de PA est réglée directement en ajustant la vitesse de broyage.

23. Procédé selon la revendication 22, dans lequel l'ajustement de la vitesse de broyage est fait au moyen d'un sélecteur intégré au broyeur.

24. Poudre cosmétique selon l'une quelconque des revendications 1 à 11, **caractérisée en ce qu'**elle contient un additif choisi parmi les pigments, les charges, les antioxydants et les liants de poudre.

**25.** Poudre cosmétique selon la revendication 24, **caractérisée en ce qu'**elle contient de la poudre de silice.

**26.** Poudre cosmétique selon la revendication 24 ou 25, **caractérisée en ce qu'**elle constitue un fard à joues ou à paupières.

**27.** Utilisation de poudre selon l'une quelconque des revendications 1 à 11, dans les revêtements, les peintures, les compositions anticorrosion, les additifs pour papier, les technologies d'agglomération de poudre par fusion ou frittage provoqué par un rayonnement pour fabriquer des objets, les gels d'électrophorèse, les matériaux composites multicouches, l'industrie de l'emballage, les jouets, le textile, l'automobile et/ou l'électronique.

**Patentansprüche**

**1.** Pulver von Polyamid PA (Homopolyamid oder Copolyamid), das sich zumindest teilweise von erneuerbaren Materialien ableitet, wobei die Teilchen eine nicht kugelförmige, unregelmäßige Form ohne scharfe Kanten und mit einem mit einem Cilas-920-Teilchengrößenbestimmungsgerät durch Laserbeugung gemessenen volumenmittleren Durchmesser im Bereich von 5 bis 20 $\mu$m aufweisen, wobei das Pulver ein gemäß der DIN-ISO-Norm 787-5 gemessenes Absorptionsvermögen im Bereich von 55 bis 110 g Öl/100 g Polyamidpulver aufweist und eine Bruchfestigkeit bei Kompaktierung des Pulvers lediglich durch Kompression unter einer Tonne im Bereich von 100 bis 600 Newton aufweist.

**2.** Polyamidpulver nach Anspruch 1, **dadurch gekennzeichnet, dass** es mindestens eines der folgenden Moleküle umfasst: 11-Aminoundecansäure, n-Heptyl-11-aminoundecansäure, Sebacinsäure, Decandiamin, eine Difettsäure, ein Fettsäuredimer und Mischungen davon.

**3.** Polyamidpulver nach Anspruch 1 oder 2, **dadurch gekennzeichnet, dass** das PA aus PA 11, PA 10.10, Copolyamiden, die mindestens eines der folgenden Monomere umfassen: 11, 10.10, 10.36, 6.10, 10.T, und Mischungen davon ausgewählt ist.

**4.** Polyamidpulver nach einem der vorhergehenden Ansprüche, **dadurch gekennzeichnet, dass** sich das Polyamid (Homopolyamid oder Copolyamid) ganz von erneuerbaren Materialien ableitet.

**5.** Pulver nach einem der vorhergehenden Ansprüche, wobei die Teilchen eine spezifische Oberfläche im Bereich von 1 bis 20 m$^2$/g, vorzugsweise 2 bis 10 m$^2$/g, vorzugsweise 3 bis 6 m$^2$/g, aufweisen.

**6.** Pulver nach einem der vorhergehenden Ansprüche, wobei die Teilchen einen volumenmittleren Durchmesser im Bereich von 5 bis 15 $\mu$m aufweisen.

**7.** Pulver nach einem der vorhergehenden Ansprüche, **dadurch gekennzeichnet, dass** sein gemäß der DIN-ISONorm 787-5 gemessenes Absorptionsvermögen im Bereich von 60 bis 90 g Öl/100 g Polyamidpulver liegt.

**8.** Pulver nach einem der vorhergehenden Ansprüche, **dadurch gekennzeichnet, dass** die Bruchfestigkeit des Pulvers bei Kompaktierung lediglich durch Kompression unter einer Tonne im Bereich von 150 bis 500 Newton liegt.

**9.** Pulver nach einem der Ansprüche 1 bis 8, **dadurch gekennzeichnet, dass** es $^{14}$C umfasst.

**10.** Pulver nach einem der Ansprüche 1 bis 9, umfassend mindestens 20 Massen-% Kohlenstoff erneuerbaren Ursprungs.

**11.** Pulver nach einem der Ansprüche 1 bis 10, umfassend mindestens 0,2.10$^{10}$ Massen-% 14C.

**12.** Verwendung von Pulver nach einem der vorhergehenden Ansprüche in kosmetischen Produkten, pharmazeutischen Produkten oder Parfümerieprodukten.

**13.** Verwendung von Pulver nach Anspruch 12 als Kompaktierungsmittel in kompakten kosmetischen Formulierungen.

**14.** Verwendung von Pulver nach Anspruch 12 oder 13 als Mattierungsmittel.

**15.** Verfahren zur Herstellung von Polyamidpulver nach einem der Ansprüche 1 bis 11, bei dem man:

- ein Pulver von Prepolymer mit einer inhärenten Viskosität von weniger als 0,5 (gemäß der ARKEMA-Methode: 0,5 g/dl in meta-Kresol bei 25°C) bei Umgebungstemperatur mahlt, wobei das Mahlen mit Hilfe einer begenstrahlmühle durchgeführt wird.

**16.** Verfahren nach Anspruch 15, bei dem das Prepolymer eine inhärente Viskosität im Bereich von 0,25 bis 0,5 (gemäß der ARKEMA-Methode: 0,5 g/dl in meta-Kresol bei 25°C) aufweist.

**17.** Verfahren nach Anspruch 15 oder 16, bei dem man dem Prepolymerpulver vor dem Mahlen pyrogen hergestelltes Siliciumdioxid zugibt.

**18.** Verfahren nach einem der Ansprüche 15 bis 17, bei dem man vor dem Mahlen:

- ein PA-Prepolymer mit einer zahlenmittleren Molmasse von weniger als 5000 g/mol und einer Viskosität von weniger als 0,5 (gemäß der ARKEMA-Methode: 0,5 g/dl in meta-Kresol bei 25°C) herstellt.

**19.** Verfahren nach einem der Ansprüche 15 bis 18, bei dem man nach dem Mahlen:

- die Viskosität der gemahlenen Prepolymerteilchen bis zu der für das Pulver gewünschten Endviskosität erhöht.

**20.** Verfahren nach einem der Ansprüche 15 bis 19, bei dem die Viskositätserhöhung durch Festphasen-Polykondensation in einem Trockner durchgeführt wird.

**21.** Verfahren nach einem der Ansprüche 15 bis 20, **dadurch gekennzeichnet, dass** bei seinen verschiedenen Schritten kein Lösungsmittel verwendet wird.

**22.** Verfahren nach einem der Ansprüche 15 bis 21, bei dem die letztendliche Teilchengröße des PA-Pulvers direkt durch Einstellung der Mahlgeschwindigkeit reguliert wird.

**23.** Verfahren nach Anspruch 22, bei dem die Einstellung der Mahlgeschwindigkeit mit Hilfe einer in die Mühle integrierten Auswahlvorrichtung erfolgt.

**24.** Kosmetisches Pulver nach einem der Ansprüche 1 bis 11, **dadurch gekennzeichnet, dass** es ein aus Pigmenten, Füllstoffen, Antioxidantien und Pulverbindemitteln ausgewähltes Additiv enthält.

**25.** Kosmetisches Pulver nach Anspruch 24, **dadurch gekennzeichnet, dass** es Siliciumdioxidpulver enthält.

**26.** Kosmetisches Pulver nach Anspruch 24 oder 25, **dadurch gekennzeichnet, dass** es ein Wangenrouge oder einen Lidschatten darstellt.

**27.** Verwendung von Pulver nach einem der Ansprüche 1 bis 11 in Beschichtungen, Anstrichmitteln, Korrosionsschutzzusammensetzungen, Papieradditiven, Techniken der Pulveragglomeration durch strahlungsinduziertes Schmelzen oder Sintern zur Herstellung von Objekten, Elektrophoresegelen, mehrschichtigen Verbundwerkstoffen, der Verpackungsindustrie, der Spielzeugindustrie, der Textilindustrie, der Automobilindustrie und/oder der Elektronikindustrie.

## Claims

**1.** Powder of polyamide PA (homopolyamide or copolyamide) derived at least partially from renewable materials, in which the particles have a nonspherical, irregular shape without sharp edges and a volume median diameter within the range of from 5 to 20 $\mu$m measured using a Cilas 920 particle sizer by laser diffraction, said powder having an absorption capacity, measured according to the DIN ISO 787-5 standard, within the range of from 55 to 110 g of oil/100 g of polyamide powder, and having a breaking strength within the range of from 100 to 600 newtons, when the powder is compacted only by compression under one tonne.

**2.** Polyamide powder according to Claim 1, **characterized in that** it comprises at least one of the following molecules: 11-aminoundecanoic acid, n-heptyl-11-aminoundecanoic acid, sebacic acid, decanediamine, a difatty acid, a dimer of a fatty acid, and mixtures thereof.

3.  Polyamide powder according to Claim 1 or 2, **characterized in that** the PA is chosen from PA 11, PA 10.10, and copolyamides comprising at least one of the following monomers: 11, 10.10, 10.36, 6.10, 10.T, and mixtures thereof.

4.  Polyamide powder according to any one of the preceding claims, **characterized in that** said polyamide, (homopolyamide or copolyamide) is entirely derived from renewable materials.

5.  Powder according to any one of the preceding claims, in which the particles have a specific surface area within the range of from 1 to 20 $m^2/g$, preferably from 2 to 10 $m^2/g$, preferably from 3 to 6 $m^2/g$.

6.  Powder according to any one of the preceding claims, in which the particles have a volume median diameter within the range of from 5 to 15 $\mu$m.

7.  Powder according to any one of the preceding claims, **characterized in that** its absorption capacity, measured according to the DIN ISO 787-5 standard, is within the range of from 60 to 90 g of oil/100 g of polyamide powder.

8.  Powder according to any one of the preceding claims, **characterized in that** breaking strength of said powder, when it is compacted only by compression under one tonne, is within the range of from 150 to 500 newtons.

9.  Powder according to any one of Claims 1 to 8, **characterized in that** it comprises $^{14}$C.

10. Powder according to any one of Claims 1 to 9, comprising at least 20% by mass of carbon of renewable origin.

11. Powder according to any one of Claims 1 to 10, comprising at least $0.2 \times 10^{-10}$% by mass of $^{14}$C.

12. Use of powder according to any one of the preceding claims, in cosmetic, pharmaceutical or perfumery products.

13. Use of powder according to Claim 12, as a compacting agent in pressed cosmetic formulations.

14. Use of powder according to Claim 12 or 13, as a matifying agent.

15. Process for preparing a polyamide powder according to any one of Claims 1 to 11, comprising:

    - milling at ambient temperature a powder of prepolymer having an inherent viscosity of less than 0.5 (according to Arkema method: 0.5 g/dl in metacresol at 25°C), said milling being carried out by means of an opposed air jet mill.

16. Process according to Claim 15, in which the Prepolymer has an inherent viscosity within the range of from 0.25 to 0.5 (according to Arkema method: 0.5 g/dl in metacresol at 25°C).

17. Process according to Claim 15 or 16, in which fumed silica is added to the prepolymer powder before it is milled.

18. Process according to any one of Claims 15 to 17, comprising, before the milling step, a step of:

    - manufacture of a prepolymer of PA having a number-average molecular mass of less than 5000 g/mol, and having a viscosity of less than 0.5 (according to Arkema method: 0.5 g/dl in metacresol at 25°C).

19. Process according to any one of Claims 15 to 18, comprising, after the milling step, a step of:

    - rise in viscosity of the milled prepolymer particles, to the final viscosity desired for the powder.

20. Process according to any one of Claims 15 to 19, in which the viscosity rise is performed by solid-phase polycondensation in a dryer.

21. Process according to any one of Claims 15 to 20, **characterized in that** its various steps do not involve a solvent.

22. Process according to any one of Claims 15 to 21, in which the final particle size of the PA powder is adjusted directly by adjusting the milling speed.

23. Process according to Claim 22, in which the adjustment of the milling speed is carried out by means of a selector integrated into the mill.

24. Cosmetic powder according to any one of Claims 1 to 11, **characterized in that** it contains an additive chosen from pigments, fillers, antioxidants and powder binders.

25. Cosmetic powder according to Claim 24, **characterized in that** it contains silica powder.

26. Cosmetic powder according to Claim 24 or 25, **characterized in that** it constitutes a face powder or an eye shadow.

27. Use of powder according to any one of Claims 1 to 11, in coatings, paints, anticorrosive compositions, additives for paper, powder agglomeration technologies by melt agglomeration or radiation sintering for manufacturing objects, electrophoresis gels, multilayer composite materials, the packaging industry, the toy industry, the textile industry, the automobile industry and/or the electronics industry.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- DE 4421454
- US 2006127424 A
- EP 1847559 A
- US 6127513 A
- FR 0753319